# EUROPEAN PATENT APPLICATION

(11) **EP 1 447 068 A1**
(43) Date of publication of application: **18.08.2004**
(21) Application number: 03425088.6
(22) Date of filing: 14.02.2003
(51) Int. Cl.: A61F 13/15, B26F 3/00, B26D 3/10, B23K 26/067, B23K 26/08, B23K 26/38

(54) **Process and device for the localised treatment of articles, for example hygienic and sanitary products**

(71) Applicant: Fameccanica.Data S.p.A., 66020 Sambuceto di S. Giovanni Teatino (Chieti) (IT)
(72) Inventor: Casuscelli, Giancarlo, 65123 Pescara (IT)
(74) Representative: Bosotti, Luciano

(57) **Abstract**

A procedure for treating, by means of a localized flow (B1, B2) such as a laser beam, a jet of pressurized water, a jet of ink, or a strand of glue, articles (A) moving in a given direction (z). The treatment involves a relative movement between the articles (A) and said at least one flow (B1, B2) along a path which, for each article (A), comprises at least one first branch and at least one second branch. The procedure comprises the operations of providing (1) at least one first flow and at least one second flow (B1, B2) for carrying out the treatment, and deflecting (2) the aforesaid flows (B1, B2) in a transverse direction (x) and, preferably, also a longitudinal direction with respect to the direction of advance (z) of the articles (A). The aforesaid flows (B1, B2) each define, for each of the articles (A), respectively the first branch and the second branch of the aforesaid path.

A preferential application is for the treatment of hygienic and sanitary products.

## Description

The present invention relates to techniques for carrying out localized treatment of articles.

In particular, the invention regards the techniques of treatment performed by means of a localized flow. Such a flow may consist, for instance, of a directed flow of radiation, such as a laser beam, which performs, on treated articles, an action of welding, perforation and/or cutting. The treatment flow may also consist - once again purely by way of example and without any intention to limit the scope of the invention in any way - of a jet of pressurized liquid, such as water used for carrying out operations of cutting according to the technology generally known as "water-jet technology", or else by a jet of printing ink, a strand of glue or other material applied on articles.

The fact of referring to a localized flow highlights the fact that the interaction between said flow and the articles treated occurs in a somewhat restricted area, for example the spot projected by a laser beam on the articles treated, with the area of interaction which moves with respect to the articles along a definite treatment path.

The invention has been developed with particular attention paid to its possible application to the treatment of hygienic and sanitary products, such as hygienic absorbent products and similar articles.

In this connection, reference may be made to the Japanese patent application P2001-145659, taken as model for the preamble of the independent claims of the present application. The Japanese document in question, in fact, describes a process for making absorbent products, using a laser apparatus for performing a function of cutting along a path of treatment defined, for each article, by at least one first branch and at least one second branch.

With respect to more traditional and consolidated applications (such as, for instance, the cutting/welding of sheet metal), the use of laser beams for the treatment of products such as hygienic and sanitary articles leads to the need to take into account critical factors of various nature.

Since the articles in question are usually articles made using materials consisting of rather thin plastic film, the supply of energy must be regulated with considerable precision (if necessary providing for a cooling function), the aim being to prevent the energy supplied by the laser beam from possibly damaging the products in an undesirable way. The said need for precision is in any case felt also when the treatment flow is of another nature (water jet for cutting, jet of printing ink, etc.) and/or when the articles treated are articles other than hygienic and sanitary ones.

Another critical factor is represented by the fact that the treatment must usually be carried out on articles (or, in any case, on elements constituting intermediate products in the production of given articles), which move in a given direction with a speed that is rather high (several hundreds of products per minute). Also this critical factor is of course felt in the presence of treatment flows of another nature and/or of articles other than hygienic and sanitary articles.

The critical factors cited above come into play both independently, and in combination with one another. It may be readily appreciated that, should the localized treatment be designed to occur - either in a continuous way or in a discontinuous way, i.e., by spots or by stretches - along a path defining, either completely or in part, the outline of an article, the fact that the article moves at a high speed generates problems of considerable difficulty.

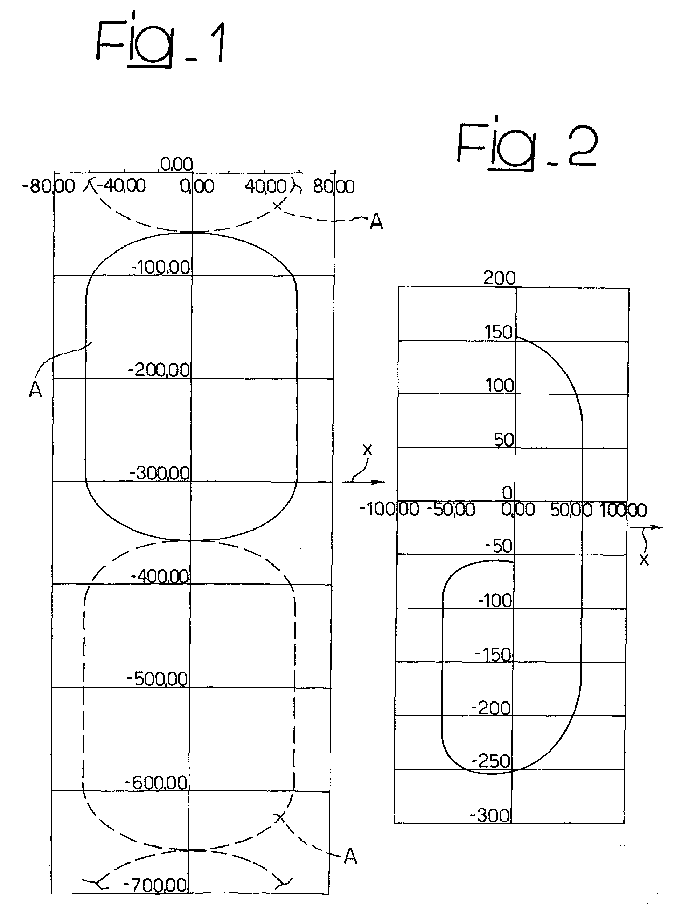

Figures 1 and 2 refer - by way of example and without any intention to limit the extent of the invention - to the possible localized application of energy by means of a laser beam (for example for purposes of welding or cutting) along the outline of articles, consisting, for example, of hygienic absorbent elements A, having an approximately rectangular shape with rounded end edges.

If it is assumed that the machine is operating on articles or elements A which are on the whole stationary, in order to cause the spot projected by the beam in the plane in which the articles A to be treated are located to follow the outline of the individual article, the beam must be subjected (according to criteria in themselves known) to an operation of scanning or deflection in two directions, i.e., a longitudinal direction z and a transverse direction x. Of course, the movement of scanning of the outline of the individual article A by the spot produced by the beam can be a relative movement, which may be obtained either by keeping the article A stationary and moving the beam or by keeping the beam stationary in space and moving the article A, or again by moving both the beam and the article A.

Should the elements A, arranged in a chain, move in the direction of the axis z represented in Figure 1, and in particular from below upwards with respect to the drawing, the path of deflection to be imparted on the laser beam becomes more complex and assumes, for the individual article A, the pattern represented in Figure 2.

Specifically, the above figure represents the effective path that the spot produced by the beam must describe to follow in a counterclockwise direction the profile of the individual element A, which moves in a longitudinal direction along the axis z. In these conditions, as long as the movement of the spot projected by the laser beam is opposite to that of the products treated, the fact that the articles A move facilitates the operation of treatment, thus rendering the corresponding path of movement quite short. Instead, in the stretch where the motion of the chain of articles A has the same direction as the movement of scanning performed by the laser spot, the path of scanning of the beam or spot lengthens considerably (note in particular, the right-hand part of Figure 2), since the beam must in effect track the articles that are moving.

This fact involves various drawbacks.

In the first place, the area "framed" by the process (i.e., the area that is to be covered by the action of deflection of the beam) must be very large and/or, in a dual way, the speed of deflection of the beam or the spot must be high to maintain the process area within acceptable dimensions.

It is then in general necessary to deactivate and reactivate the energy flow of the beam within very short time intervals to enable repositioning once processing of an article A is completed in order to operate on the next element in the chain of articles treated.

The plants for the fabrication of hygienic and sanitary products, such as hygienic absorbent products for ladies and similar products, operate at rates that can arrive at values of the order of 1500-2000 articles per minute. Albeit resorting to systems for managing movement of the beam that are very complex and costly (large and fast), the corresponding solution, obtained according to the criteria of the known art, ends up by falling within the limits of impraticability.

The purpose of the present invention is to provide a solution capable of overcoming the drawbacks intrinsic in the solutions according to the known art.

According to the present invention, said purpose is achieved thanks to a procedure having the characteristics referred to specifically in the claims that follow.

The invention also relates to the corresponding device.

Essentially, the solution according to the invention envisages organizing the treatment flow (laser beam, jet of water at high pressure, ink jet, etc.) into at least one first flow and at least one second flow. To each of the (sub)flows in question there is entrusted the treatment of a stretch of the application path envisaged i.e., with reference to the example already illustrated in Figures 1 and 2, of the outline of the individual articles A. The stretch in question is chosen so as to correspond to the conditions in which the movement of the articles cooperates in a favourable way to creating the relative movement of the localized treatment flow, the aim being to prevent the treatment flow having to track the articles, as occurs in the right-hand part of Figure 2.

According to a particularly preferred embodiment, there is envisaged the use of two flows: the first flow has the job of scanning a semi-profile - for example the left-hand side, in a longitudinal direction - of an article and then the opposite semi-profile - i.e., the right-hand side, once again in a longitudinal direction
- of the next article, and so forth; the second flow has the job of scanning the complementary semi-profiles, i.e., the ones not treated by the first flow.

According to a preferred embodiment, the two flows are governed by a processing unit that provides, according to predetermined laws, for the synchronization of the deflection of the two flows and of the movement of the articles undergoing treatment.

The aforesaid movement of deflection may occur both in a direction that is exclusively transverse with respect to the direction of advance of the articles, and - according to a preferred embodiment - so as to present also a component of movement in a direction parallel to the direction of advance of the articles.

The movement of deflection in a direction parallel to the direction of advance of the articles can occur both in the same direction and in the opposite direction to that of advance of the articles, the aim being to maintain constant (thanks to the superposition of the two orthogonal components of movement, in the direction of advance of the articles treated and in a transverse direction) the speed with which the treatment flow describes its path on the articles that are treated.

The corresponding paths of deflection are closed figures, substantially resembling Lissajous figures (the other than complete analogy deriving from the fact that, in the present case, the compound motions are not exactly forms of harmonic motion).

The invention will now be described, purely by way of non-limiting example, with reference to the annexed drawings, in which:
- Figures 1 and 2 have already been described previously;
- Figure 3 is a side elevation of a system operating according to the invention; and
- Figures 5 to 8 illustrate, according to modalities substantially similar to a those adopted in Figures 1 and 2, the operating criteria of the solution described herein.

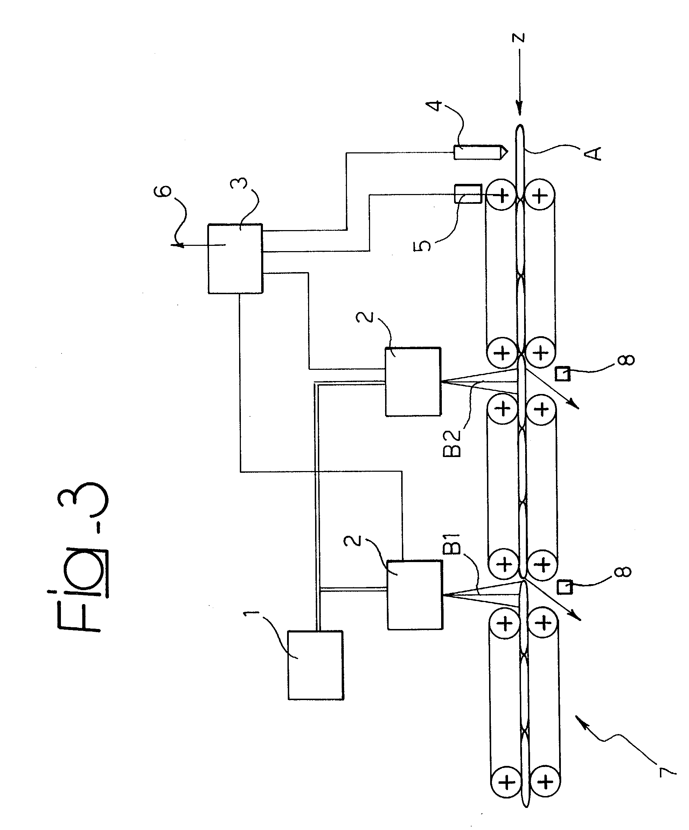

In the general diagram of Figure 3, the reference number 1 designates a source of a treatment flow that is to be applied on articles A, which move (with a speed that will be assumed herein, purely by way of example, constant and directed from right to left, as viewed in Figure 3) in a direction generically designated by z.

The source 1 may consist, for example, of a laser source for generating a laser beam, which, projected on the articles A, forms thereon an areola or interaction spot. Said areola or interaction spot is designed to perform, on the articles A themselves, - according to criteria in themselves known - an action of welding or cutting along a predetermined path, corresponding, for instance, to the outline of the articles A themselves.

Even though the description provided in what follows will refer preferentially to the use of a laser source designed to be employed for performing an action of welding and/or cutting on the articles A, it will be appreciated that the solution described herein may be applied in any context in which it is necessary to carry out local treatment on moving articles, operating according to a given path.

Consequently, the source 1 may consist, for example, of a source of glue, an ink pump, or a source of a jet of pressurized water of the type used in cutting systems commonly referred to as "water-jet" cutting systems. It is therefore, in general, a source of flow that can be applied on the articles A so as to perform, on the articles A themselves, a localized treatment.

The treatment can be performed in a continuous way, or else in a discontinuous way, i.e., by spots or by stretches, so as to give rise, for example, to a spot weld or a perforation by spots or by stretches (so-called pricking or dinking).

In the example of embodiment illustrated herein (which, it is recalled, is intended merely as nothing more than an example), the laser beam produced by the source 1 is sent to two beam-deflection systems 2.

Each system 2 is able to send back in the direction of the articles A a respective portion (B1 or B2) of the laser beam received from the source 1, imparting on said respective portion of beam a movement of deflection which is chiefly in a transverse direction with respect to the direction of movement of the articles A (axis x in Figures 1, 2 and 4 to 7), as well as - in a preferred way - also in the direction of said movement (axis z in the said figures).

For example, the source 1 may be a CO₂ or YAG laser source (or also a semiconductor laser source and/or fibre-optic laser source, possibly having associated thereto amplifier devices which bestow the necessary power on the beam generated). The beam-deflection systems 2 may consist of commercially available devices, such as for example the optical scanning head of the scanning model No. HPM10A, manufactured by General Scanning Inc., of Watertown (U.S.A.), or else by the products Harryscan 25 or Powerscan 33, manufactured by the company Scanlab (Germany).

In this case, the laser beam or ray at output from the source 1 is received from an inlet opening of each deflection system 2 and deviated by means of a pair of mirrors with fast-recovery galvanometric movement, for any axis z and x.

Each of the laser beams or rays B1 or B2 coming from each beam-deflection system 2 is thus able to arrive on the articles A in the form of a localization spot or an areola with a degree of focusing that is selectively determinable (in a way in itself known) by acting on the beam-deflection systems 2.

The range of action of each system 2 in the plane of the articles A may be a square or a rectangle, which can range, in dimensions, typically from between approximately 100 x 100 millimetres and approximately 500 x 500 millimetres, respectively, along the axis x and along the axis z, according to the focusing lens used in the system 2 and the distance of this from the plane in which the articles are located (processing plane).

It will be appreciated, on the other hand, that, as an alternative to the solution illustrated herein (use of one source 1 and two beam-deflection systems 2), the solution described is suitable for being implemented also using, instead of each deflection system, a respective source, which generates a corresponding treatment flow B1 or B2. In the same way, the number of treatment flows used (and, correspondingly, the number of beam-deflection systems and/or of sources used for their generation) may be more than two.

Furthermore, the small dimensions of the scanning window imparted on the flows B1 and B2 makes available corresponding generation/scanning systems 2, either combined or set alongside one another, instead of being cascaded together, as illustrated in Figure 3.

Returning to the diagram of Figure 3, the reference number 3 designates an electronic control unit (such as, for example, a dedicated computer card), which supervises operation of the system, controlling the action of deflection performed by the systems 2 on the beams B1 and B2. This occurs according to signals supplied by sensors, such as a sensor 4, which detects the position of the articles A, and a sensor 5 consisting of a speed sensor, which detects the speed of advance of the articles A along the axis z.

The reference number 6 designates, in general, an interface to a line controller, which supervises operation of the system, in which there is inserted the device represented in Figure 3.

The reference number 7 designates a conveying system used for feeding the articles A in the direction z. Advantageously, the system in question consists of a motor-driven-belt system comprising, for example, pairs of motor-driven belts set one above the another, the conveying branches of which act one on the bottom face and the other on the top face of the articles A included in the flow.

Of course, the conveying system 7 may be of a type different from the one illustrated and may envisage, for example, conveyors designed to operate exclusively on the bottom face of the articles A, supporting their flow in the direction of the axis z.

In a preferred way, the beam-deflection systems 2, which generate the beams B1 and B2 which are to operate on the articles A, are located in such a way that their working area comes to correspond to a discontinuity in the conveyor 7.

Once again in a preferred way, provided at the discontinuities of the conveyor 7 are devices 8 (for example, air-suction devices) for elimination of any possible processing scrap or waste.

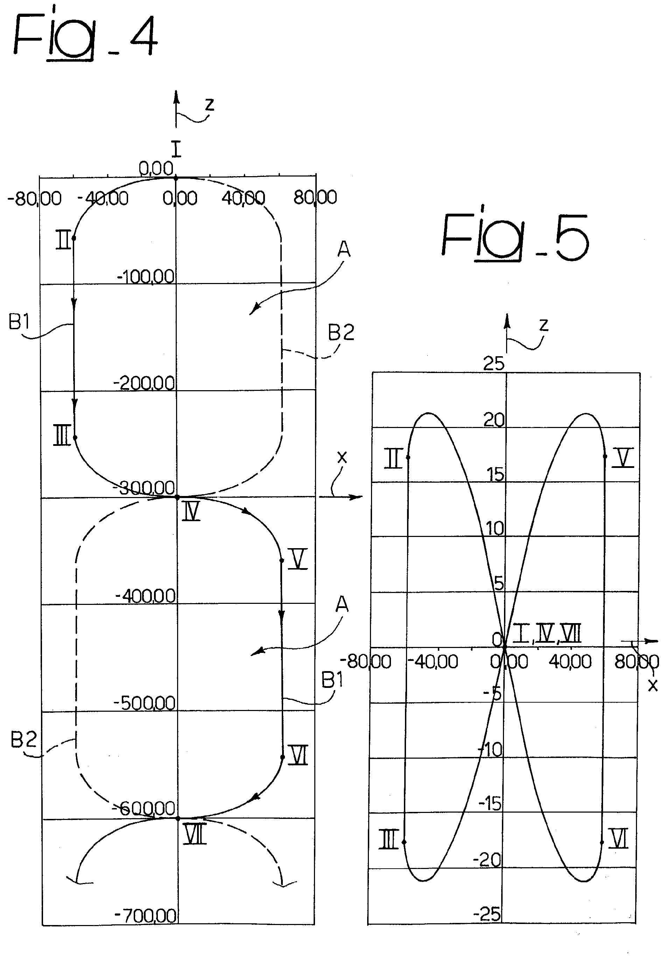

Figures 4 and 5 illustrate the criteria that may of be adopted in a system, such as the one illustrated in Figure 3, for carrying out a localized treatment (such as, a laser-welding or laser-cutting operation) on articles A having an outline identical to the outline of the article schematically represented in Figure 1.

Basically, the solution described herein envisages that on each of the flows or beams B1 and B2 generated by the system 2 there are imparted respective deflection paths such as to cause the respective beam or flow not to define completely the outline of each individual article A, but only part thereof.

In the solution described herein, it is envisaged that each flow B1, B2 is designed to describe a respective portion of the aforesaid outline. Each portion is defined in such a way that the movement of scanning of the article by the beam, and the consequent definition of a corresponding part of outline, advantageously exploits the superposition of the movement of advance of the articles along the axis z and the movement of deflection (both transverse, along the axis x, and longitudinal, along the axis z) imparted on the beams B1 and B2.

In a preferred way, the roles played by the two beams B1 and B2 are inverted from one article A to the next article. The diagram of Figure 4 shows, in fact, that on each of the beams B1 and B2 there is imparted (in a complementary way, and hence with directions that are opposite along the axis x) a transverse movement of deflection such as to cause the spot projected by each beam on the articles A undergoing treatment (laser cutting/laser welding, in the example illustrated herein) to correspond to a zigzag path or approximately sinusoidal path.

By operating in this way, for a given article A, the beam B1 carries out welding/cutting of the right-hand side, whilst the beam B2 carries out welding/cutting of the left-hand side, and then, for the next article, the beam B1 carries out welding/cutting of the left-hand side, whilst the welding/cutting of the right-hand side is now performed by the beam B2. This is followed then, in the next article in the chain, by the situation in which the beam B1 again carries out welding/cutting of the right-hand side and the beam B2 carries out welding/cutting of the left-hand side, and so forth. Of course, the definitions "right" and "left", referring to the principle median axis of the articles A are to be understood in an arbitrary way and can thus be exchanged with one another.

Once again, even though the paths traversed by the beams B1 and B2 represented in Figure 4 (and, in a corresponding way the deflection paths represented in Figure 5), are symmetrical with respect to the principal central axis of the articles A, this characteristic of symmetry does not constitute in any way a basic requisite of the invention.

In fact, the paths designated herein by B1 and B2 (in what follows, these paths will in fact be equated with the beams which generate them in order to simplify the description) are constituted by winding paths comprising half-waves which extend in an alternating and symmetrical way with respect to the longitudinal principal axis of the article A (i.e., the axis z of the drawings); the said paths could, however, have an asymmetrical extension with respect to the aforesaid principal axis.

For evident reasons of continuity of performance of the action of deflection of the beam, the embodiment of the invention currently preferred envisages that each of the paths B1 and B2 will present a zigzag pattern, with half-waves arranged alternately on one side and the other side with respect to the axis z, so that the paths defined by the first flow B1 and by the second flow B2 cross one another at said principal axis.

Also this characteristic is not, however, imperative, since the solution could be implemented with a path B1 comprising all half-waves situated on one side with respect to the principal longitudinal axis of the articles A and with a path B2 comprising, in a complementary way, the half-waves situated on the other side with respect to the principal longitudinal axis of the articles A.

In this case, the paths B1 and B2 would present a pattern that substantially resembles the pattern of a rectified sinusoidal current. Albeit practicable and comprised in the scope of the invention, this solution is not considered at the moment particularly preferred in that it entails imposing sharp changes of direction on the movement of deflection of the individual beams. Such changes of direction occur at the two cusps which, in Figure 4, correspond to the cross-over point of the paths B1 and B2.

Once again it should be recalled that, instead of resorting to two (sub)beams defining two complementary paths B1 and B2, the solution described herein can be implemented also using a greater number of beams.

The solution that envisages recourse to two beams represents, however, a preferred solution in that it represents an ideal synthesis between quality of results achieved and simplicity of implementation.

To understand exactly the meaning of Figure 5, it is necessary, in the first place, to note that the diagram of Figure 4 corresponds to the two paths described in the plane of the articles A by the two treatment flows or beams B1 and B2 coming from the source 1.

The "reference system" of Figure 4 thus consists of the row or chain of articles A, which advance along the conveyor 7 and in which the beams produced by the beam-deflection systems 2 describe the paths B1 and B2.

The reference system of the diagram of Figure 5 is, instead, a stationary reference system, with the device structure represented in Figure 3. The diagram of Figure 5 can therefore be viewed as representative of the path that the spot generated by each of the beams coming from the systems 2 follows in the area of separation between successive branches of the conveying system 7.

At least in principle, paths such as the paths B1 and B2 of Figure 4 could be defined each by one of the beams or rays produced by the device 2 as a result of a pure and simple movement of deflection oriented along the axis x, i.e., in a transverse direction with respect to the axis z.

In other words, observing Figures 4 and 5 in a coordinated way, the "half-waves" of the path B1 which are on the left with respect to the axis z could be obtained by simply deflecting to the left (negative values of the axis x of Figure 5) the flow or beam each time considered. The half-waves of the path B1, which are on the right with respect to the axis z, could also be generated as a result of a pure and simple movement of deflection to the right in the diagram of Figure 5 (positive values of x in the same Figure 5). In this case, the diagram of Figure 5 would be reduced to a pure and simple horizontal segment.

In a preferred way, instead of being a pure and simple horizontal segment, the deflection path represented in Figure 5 is a closed path with a butterfly-shaped pattern, which substantially resembles a Lissajous figure: in the preferred embodiment of the invention, each of the beams or rays B1 and B2 produced by the systems 2 is, in fact, subjected not only to a deflection along the axis x (i.e., in a transverse direction with respect to the direction of advance of the articles A), but also to a movement of deflection in the direction of the axis z.

Limiting our description to the path B1 (but the same argument can, with the necessary changes made, be applied to the path B2), in the path B1 of Figure 4 there are identified seven points, designated by Roman numerals, from I to VII. Similar indications are given in the diagram of Figure 5 to identify the corresponding points.

From a comparison of the diagrams of Figures 4 and 5, it will therefore be appreciated as described in what follows.

To make half of the rounded front edge of the article A corresponding to the path between the points I and II of Figure 4, the corresponding beam (in the example this is the beam B1, but the situation is altogether equivalent for the beam B2 if the next article A in the chain or flow of articles A is considered) does not undergo, in the corresponding system 2, a pure and simple movement of deflection along the axis x. There is in fact imparted on the beam in question the curved deflection path comprised between the points I and II in Figure 5.

The said path corresponds to a movement of deflection which, in addition to a first component along the axis x, has also a second component in the direction of the axis z, which leads the spot generated by the beam in question to follow the articles A in their movement along the axis z (from below upwards as viewed Figure 4). In the absence of said movement to be followed, the path in question would end up being much closer to the axis z, with the result of giving rise to an article A with a front edge having a markedly curved pattern, if, indeed, not a sharp cusp-like pattern.

Furthermore, the deflection path comprised between the points I and II of the diagram of Figure 5 is determined (according to geometrical criteria of composition, which are in themselves known, taking into account the speed of advance of the articles along the axis z, as well as the error of parallax deriving from the distance existing between the systems 2 and the plane in which the articles A move) in such a way as to cause the spot projected by the beam B1 to traverse the respective portion of outline of the article A at a constant speed, ensuring that the energy supplied by the laser beam will be applied in an equally constant way in terms of energy per unit length of the path. In this way there is avoided both the undesirable overheating of the material (with the risk that an action of welding will result in an action of cutting), and an equally undesirable insufficient heating (with the risk that an intended action of cutting will be limited to an action of welding).

Once the point designated by II in Figures 4 and 5 has been reached, the beam is kept in a fixed position with respect to the axis x, causing the corresponding spot to define the rectilinear stretch of the path B1 comprised between the points designated by II and III. In the meantime (as may be seen more clearly from the diagram of Figure 5), the beam is subjected to a movement of deflection backwards along the axis z, which causes it to reach the point III of Figure 5 in conditions of substantial symmetry in regard to the point II with respect to the axis x.

Also in this stretch of path, the action of deflection is determined (once again taking into account the speed of advance of the articles along the axis z and the parallax error deriving from the distance existing between the systems 2 and the plane in which the articles A move) in such a way as to cause the spot projected by the beam B1 to traverse the respective portion of outline of the article A at a constant speed, ensuring that the energy supplied by the laser beam will be applied in an equally constant way in terms of energy per unit length of the path.

Here onwards then, to make half of the rounded rear edge of the article A corresponding to the path between the points III and IV of Figure 4, the beam (referring once again to the beam B1) does not undergo, in the corresponding system 2, a pure and simple movement of deflection along the axis x aimed at bringing it back towards the axis z.

The return in the direction of the axis z is in fact achieved by imparting on the beam B1 the curved deflection path comprised between the points III and IV of Figure 5. Also in this case, as in the case of the front edge of the article A, the said path corresponds to a movement of deflection which, in addition to a first component along the axis x, also has a second component in the direction of the axis z, which once again leads the spot generated by the beam in question to follow or track the articles A in their movement along the axis z (from below upwards, as viewed in Figure 4). In the absence of said movement to be followed, the path in question would end up being much closer to the axis z, with the result of giving rise to an article A with a rear edge presenting a sharp or cusp-like pattern, resembling the tail of a drop of water.

Once again, the deflection path comprised between the points III and IV of the diagram of Figure 5 is determined (once more taking into account the speed of advance of the articles along the axis z and the parallax error deriving from the distance existing between the systems 2 and the plane in which the articles A move) in such a way as to cause the spot projected by the beam B1 to traverse the respective portion of outline of the article A at a constant speed, ensuring that the energy supplied by the laser beam will be applied in an equally constant way in terms of energy per unit length of the path.

Now that the beam has been brought back to the centre of the diagram of Figure 5 (corresponding to points I, IV and VII, identified on the path B1 of Figure 4), the same law of deflection is followed in an exactly symmetrical and specular way for describing the complementary part of the path B1 corresponding to the next article A.

The references IV, V, VI and VII designate, in the right-hand part of the diagram of Figure 5, the points corresponding to the points IV, V, VI and VII of the path B1 of Figure 4.

It will be moreover appreciated that the example represented in Figure 4 envisages that the various articles A are arranged in the form of a chain of articles A that are strictly consecutive with respect to one another, i.e., with the rear edge of one article A touching (on the axis z) the front edge of the next article A, which causes the paths defined by the two beams B1 and B2 to cross over one another at the longitudinal axis z of the articles A.

Albeit preferred, the latter solution is not imperative. It is in fact possible also to make articles A set at a distance apart from one another, envisaging that, in the stretches comprised between successive articles, each beam or ray will be kept oriented in such a way that the spot projected in the plane of the articles A will follow a rectilinear path along the axis z. In terms of action of deflection, said result can be obtained by keeping the beam in the central point of the diagram of Figure 5 (zero-deflection value both with respect to the axis x and with respect to the axis z).

It will be appreciated that the rules which enable definition of the deflection diagram represented in Figure 5 according to the pattern of the paths B1 and B2 (in practice, the shape of the articles A) are generated in the context of the processing unit 6 according to the signals supplied by the sensors 4 and 5 on the basis of elementary laws of composition of motion.

In practice, the operation of the system described herein is based upon the phase control of the products or articles A and on the control of the spot that is to describe the profile defined in the same time interval employed by the product to traverse a space corresponding to its length.

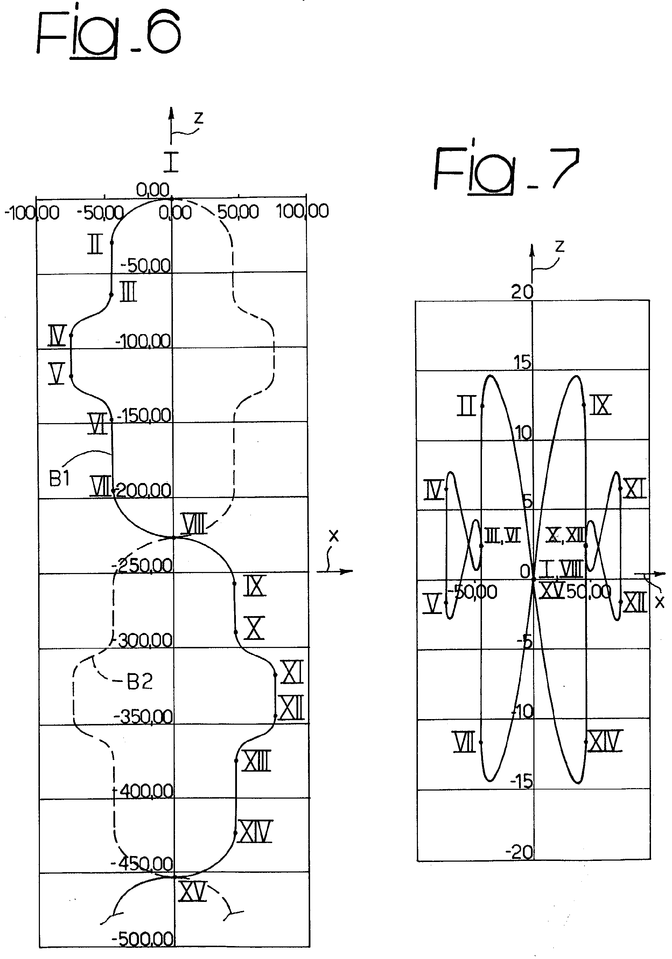

For example, Figures 6 and 7 reproduce, with a formalism that is substantially similar to the one of Figures 4 and 5, the pattern of a path homologous to the path B1 and the pattern of the corresponding deflection path in the case of an article having a more complex shape than the shape of the articles A represented in Figure 4.

For immediate reference, the articles A, to which Figures 6 and 7 refer, are so-called hygienic absorbent pads or liners provided with side flaps, the said flaps being located in positions that are slightly asymmetrical with respect to the longitudinal development of the articles along the axis z.

Also in this case, designated by progressive Roman numerals from I to XV are successive points of the path B1 of Figure 6, as well as the corresponding points of the deflection path of Figure 7.

Of course the solution according to the invention may be applied to paths of a different type, also ones having a development that is more complex than the one illustrated herein.

By adopting the technique described herein, it is possible to achieve a number of considerable advantages.

In the first place, the speed necessary for the beam or ray to scan or explore the articles or products becomes much lower and is in effect comparable to the speed of conveying of the products themselves, thus resulting easily achievable by current systems. In addition to this, since it is possible to operate at a low speed, it is possible to use flows (beams, strands, or jets) the supply of energy of which and/or the supply of material that is to be transferred onto the articles A is less.

The area "framed" by the process (i.e., in practice, the area in the framework of which the action of deflection performed by the devices 2 of Figure 1 is exerted) is reduced drastically. For example, it is possible to have a deflection window of the width of just 42 millimetres for treating a product having a length of 30 centimetres.

The figure identified by the deflection path (Figures 5 and 7) moreover presents the additional advantage of not changing with the variation in the speed of the product. Said figure is in fact the one that minimizes the accelerations necessary for traversing the profiles with the spot. In fact, in conditions of synchronization corresponding to the closed figure, the speed vector corresponding to the end of a product is the same as the one for the start of the next product (at the point of origin of the cartesian space of Figures 5 and 7).

What has been described above results in the effective possibility of carrying out the process using currently existing technologies, which means a considerable saving in terms of components used (less power and lower performance levels required), as well as achieving a greater reliability due to the lower stresses on of the components, thus ensuring a higher quality of the process and of the corresponding results.

Of course, without prejudice to the principle of the invention, the details of implementation and the embodiments may vary widely with respect to what is described and illustrated herein, without thereby departing from the scope of the present invention, as defined in the annexed claims.

## Claims

1. A process for treating, by means of at least one localized flow (B1, B2), articles (A) moving in a given direction (z), the treatment involving a relative movement between said articles (A) and said at least one flow (B1, B2) along a path which, for each article (A), comprises at least one first and at least one second branch, **characterized in that** it comprises the operations of:
- providing (1) at least one first flow and at least one second flow (B1, B2) for carrying out said treatment,
- deflecting (2) said at least one first flow and at least one second flow (B1, B2) in a transverse direction (x) with respect to said given direction (z), said at least one first flow and at least one second flow (B1, B2) each defining, for each of said articles (A), said first branch and said second branch of said path, respectively.

2. The process according to Claim 1, **characterized in that** it further comprises the operation of deflecting said at least one first flow and at least one second flow (B1, B2) also in said given direction (z), the superposition between said deflection in a transverse direction (x) with respect to said given direction (z) and of the deflection in said given direction (z) giving rise, for each between said at least one first beam and at least one second beam, to a deflection path.

3. The process according to Claim 2, **characterized in that** said deflection path substantially resembles a Lissajous figure.

4. The process according to any one of the preceding claims, **characterized in that** it comprises the operations of:
- feeding said articles (A) in sequence in said given direction; and
- deflecting said at least one first flow and at least one second flow (B1, B2) causing each of said first and second flows (B1, B2) to describe, respectively :
- the first branch of the path for said article (A) and the second branch of the path for the next article (A) in said sequence; and
- the second branch of said article (A) and the first branch of the path for said next article in said sequence.

5. The process according to Claim 4, **characterized in that** said first branch and said second branch of said path for each article (A) are localized on opposite sides of a principal axis (z) of said articles (A) oriented in said given direction, the deflection imparted on said at least one first flow (B1) and at least one second flow (B2) causing each between said at least one first flow and at least one second flow to define, with respect to said articles, a path that is on the whole sinuous, with successive bends localized, one on one side and one on the other side with respect to said principal axis (z) of the articles (A).

6. The process according to Claim 5, **characterized in that** the paths defined on said articles (A) by said at least one first flow (B1) and at least one second flow (B2) cross one another at said principal axis.

7. The process according to Claim 2, **characterized in that** said deflection in said given direction comprises at least one first portion (I, II; III, IV) in the same direction as the direction of movement of said articles and at least one second portion (II, III) in the opposite direction with respect to the direction of movement of said articles.

8. The process according to any one of the preceding claims, **characterized in that** it comprises the operation of selecting said treatment flow in the group consisting of: a laser beam, a jet of pressurized liquid, a jet of ink, and a strand of material to be applied on said articles.

9. A device for treating, by means of at least one localized flow (B1, B2), articles (A) moving in a given direction (z), the treatment involving a relative movement between said articles (A) and said at least one flow (B1, B2) along a path which, for each article (A), comprises at least one first branch and at least one second branch, **characterized in that** it comprises the operations of:
- a source (1) of at least one first flow and at least one second flow (B1, B2) carrying out said treatment,
- a deflection system (2) deflecting said at least one first flow and at least one second flow (B1, B2) in a transverse direction (x) with respect to said given direction (z), said at least one first flow and at least one second flow (B1, B2) each defining, for each of said articles (A), respectively, said first branch and said second branch of said path.

10. The device according to Claim 9, **characterized in that** said deflection system (12) is configured for deflecting said at least one first flow and at least one second flow (B1, B2) also in said given direction (z), the superposition between said deflection in a transverse direction (x) with respect to said given direction (z) and the deflection in said given direction (z) giving rise, for each between said at least one first beam and at least one second beam, to a deflection path.

11. The device according to Claim 10, **characterized in that** said deflection system (12) is configured for deflecting said at least one first flow and at least one second flow (B1, B2) according to a closed deflection path that substantially resembles a Lissajous figure.

12. The device according to any one of the preceding Claims 9 to 11, **characterized in that** it comprises a conveyor (7) for feeding said articles (A) in sequence in said given direction, and **in that** said deflection system (12) is configured for deflecting said at least one first flow and at least one second flow (B1, B2), causing each of said first flow and second flow (B1, B2) to describe, respectively:
- the first branch of the path for one said article (A) and the second branch of the path for the next article (A) in said sequence; and
- the second branch of said article (A) and the first branch of the path for said next article in said sequence.

13. The device according to Claim 12, **characterized in that** said first branch and said second branch of said path for each article (A) thus resulting localized on opposite sides of a principal axis (z) of said articles (A) oriented in said given direction, said deflection system (12) is configured for deflecting said at least one first flow (B1) and at least one second flow (B2), causing each between said at least one first flow and at least one second flow to define, with respect to said articles, a path that is on the whole sinuous, with successive bends located, respectively, on one side and on the other side with respect to said principal axis (z) of the articles (A).

14. The device according to Claim 13, **characterized in that** said deflection system (12) is configured for deflecting said at least one first flow and at least one second flow (B1, B2), causing the paths defined on said articles (A) by said at least one first flow (B1) and at least one second flow (B2) to cross one another at said principal axis.

15. The device according to Claim 10, **characterized in that** said deflection system (12) is configured for deflecting said at least one first flow and at least one second flow (B1, B2) in said given direction according to at least one first portion (I, II; III, IV) in the same direction as the direction of movement of said articles and at least one second portion (II, III) in the opposite direction with respect to the direction of movement of said articles.

16. The device according to any one of Claims 9 to 15, **characterized in that** said source (1) of flow is chosen in the group consisting of: a laser-beam source, a pressurized-liquid-jet source, an ink-jet source, and a source of a strand of material to be applied on said articles (A).
